# EUROPÄISCHE PATENTSCHRIFT

(11) **EP 3 181 541 B1**
(45) Veröffentlichungstag und Bekanntmachung des Hinweises auf die Patenterteilung: **29.08.2018**
(21) Anmeldenummer: 15400057.4
(22) Anmeldetag: 18.12.2015
(51) Int. Cl.: C07C 29/76, C07C 31/04, C07C 29/151

(54) **VERFAHREN UND VORRICHTUNG ZUR WIEDERGEWINNUNG VOM METHANOL**
METHOD AND DEVICE FOR THE RECOVERY OF METHANOL
PROCEDE ET DISPOSITIF DE RECUPERATION DE METHANOL

(43) Veröffentlichungstag der Anmeldung: 21.06.2017
(73) Patentinhaber: L'Air Liquide Société Anonyme pour l'Etude et l'Exploitation des Procédés Georges Claude, 75007 Paris (FR)
(72) Erfinder: Gronemann, Veronika, 61184 Karben (DE); Huder, Karin, Dr., 60435 Frankfurt am Main (DE); Oelmann, Tobias, 60433 Frankfurt am Main (DE)
(74) Vertreter: Dropsch, Holger

(56) Entgegenhaltungen:
- EP-A1- 2 168 938
- EP-B1- 0 802 893
- DATABASE WPI Week 198127 Thomson Scientific, London, GB; AN 1981-48488D XP002758365, & JP S56 55324 A (TOYO ENG CORP) 15. Mai 1981 (1981-05-15)
- DATABASE WPI Week 200142 Thomson Scientific, London, GB; AN 2001-392588 XP002758366, & JP 2001 039911 A (MITSUBISHI GAS CHEM CO INC) 13. Februar 2001 (2001-02-13)
- DATABASE WPI Week 198116 Thomson Scientific, London, GB; AN 1981-28269D XP002758367, & JP S56 20528 A (TOYO ENG CORP) 26. Februar 1981 (1981-02-26)

## Beschreibung

### Gebiet der Erfindung

Die vorliegende Erfindung betrifft ein Verfahren zur Wiedergewinnung von Methanol aus Prozessabgasen der Methanolsynthese. Weiterhin umfasst die Erfindung eine Vorrichtung zur Durchführung dieses Verfahrens.

### Stand der Technik

Verfahren zur Herstellung von Methanol durch katalytische Umsetzung von Wasserstoff und Kohlenstoffoxide enthaltendem Synthesegas sind der Fachwelt seit langer Zeit bekannt. So werden in Ullmann's Encyclopedia of Industrial Chemistry, Sixth Edition, 1998 Electronic Release, Kapitel "Methanol", Unterkapitel 5.2 "Synthesis" verschiedene Grundverfahren zur Herstellung von Methanol beschrieben.

Ein moderneres, zweistufiges Verfahren zur Herstellung von Methanol ist beispielsweise aus der EP 0 790 226 B1 bekannt. Das Methanol wird in einem Kreisprozess hergestellt, bei dem eine Mischung von frischem und teilweise reagiertem Synthesegas zunächst einem wassergekühlten Reaktor und dann einem gasgekühlten Reaktor zugeführt wird, in welchen das Synthesegas jeweils an einem kupferbasierten Katalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280°C vorgewärmt wird, bevor es in den ersten Synthesereaktor eingebracht wird. Ein Teil des zurückzuführenden Synthesegases wird als Spülstrom (sog. Purge) aus dem Verfahren entfernt, um zu verhindern, dass sich Inertkomponenten innerhalb des Synthesekreislaufes anreichern. Diese Maßnahme wird auch in der deutschen Offenlegungsschrift DE 2934332 A1 und der europäischen Patentanmeldung EP 1016643 A1 gelehrt.

Der aus dem Synthesekreislauf ausgeleitete Purgegasstrom ist noch mit signifikanten Anteilen an Methanol beladen. Dasselbe gilt für andere Abgasströme, die innerhalb der Methanolsynthese und der Aufarbeitung der Rohprodukte erhalten werden, wie beispielsweise Abgasen aus Methanol-Entspannungsbehältern oder Abgasen aus Lagertanks für Rohmethanol, Methanol-Wasser-Gemischen oder Reinmethanol.

Methanol-Lagertanks werden häufig als Festdachtanks ausgeführt, wobei zur Inertisierung des nicht mit Methanol ausgefüllten Innenvolumens dieses häufig mit Stickstoff gespült bzw. ausgefüllt wird. Die Stickstoffatmosphäre sättigt sich dabei mit Methanol. Bei Beladung der Tanks oder durch Tankatmung, beispielsweise durch Sonneneinstrahlung, wird mit Methanol beladener Stickstoff aus dem Tank ausgeleitet, um einen Überdruck im Tank zu vermeiden.

Die Methanol-Anteile solcher Abgase sind aufgrund des niedrigen Siedepunkts des Methanols von 65 °C bei Umgebungsdruck durchaus bedeutend, da die Abgase in der Regel bei der jeweiligen Temperatur mit Methanoldampf gesättigt sind. So beträgt unter Sättigungsbedingungen die Methanolkonzentration in einem Abgasstrom mit einer Temperatur von 42 °C rund 33 Vol.-%.

Angesichts dieser Methanolgehalte ist es plausibel, dass die Wiedergewinnung des Methanols aus den Abgasen einen wichtigen Beitrag zur Wirtschaftlichkeit des Methanolsyntheseverfahrens liefert. Andererseits ist es auch aus Umweltschutzgründen nicht vertretbar, vor dem Hintergrund der hohen Toxizität des Methanols Abgase mit derart hohen Methanolgehalten ohne Nachbehandlung an die Umwelt abzugeben. Schließlich bereiten hohe Methanolgehalte in Abgasen auch bei ihrer Weiterverarbeitung Probleme, da Methanol auskondensieren und so beispielsweise Gasbrenner beschädigen kann.

Aufgrund der hohen Wasserlöslichkeit des Methanols hat sich als Abscheidemethode die Wäsche der Abgase mit Wasser als Wachmittel bewährt. So lehrt die offengelegte europäische Patentanmeldung EP 2 168 938 A1 die Wiedergewinnung von Methanol aus Abgasen der Rohmethanoldestillation durch Wasserwäsche in einer Gegenstromkolonne. Auch die europäische Patentschrift EP 0 802 893 B1 beschreibt die Abtrennung und Wiedergewinnung von Methanol aus Abgasen der Rohmethanoldestillation durch Wasserwäsche.

Das US-Patent 5 346 593 A beschreibt eine Destillationskolonne zur Gewinnung von Reinmethanol, die an ihrem Kopf mit einer Wasserwaschstufe ausgestattet ist und zur Abscheidung von Methanolresten aus einem die Kolonne verlassenden Abgasstrom dient.

Schließlich offenbart die europäische Patentschrift EP 0 009 385 B1 die Abtrennung von Methanol aus dem aus dem Synthesekreislauf ausgeleiteten Purgegasstrom mittels Wasserwäsche und die Wiedergewinnung des abgetrennten Methanols mittels Destillation.

Insgesamt ist jedoch festzustellen, dass trotz des oben diskutierten Stands der Technik weiterhin Bedarf an optimierten Verfahren und Vorrichtungen zur Abscheidung und Wiedergewinnung von Methanol aus verschiedenen Abgasströmen besteht, die innerhalb der Methanolsynthese und der Aufarbeitung der Rohprodukte erhalten werden. Insbesondere hinsichtlich der Integration solcher Verfahrensstufen in das komplexe Fließschema der Aufarbeitung des Rohmethanols, der Wiedergewinnung von Methanol aus mehrerer der genannten Abgasarten sowie hinsichtlich der verfahrenstechnischen Ausgestaltung der verwendeten Apparate besteht hier noch Optimierungsbedarf.

### Beschreibung der Erfindung

Vor diesem Hintergrund besteht die Aufgabe der vorliegenden Erfindung darin, ein Verfahren und eine Vorrichtung bereitzustellen, bei dem bzw. durch die eine effiziente Wiedergewinnung von Methanol aus Prozessabgasen der Synthese und Weiterverarbeitung von Methanol sichergestellt wird.

Diese Aufgabe wird durch ein Verfahren mit den Merkmalen des Anspruchs 1 und durch eine Vorrichtung mit den Merkmalen des Anspruchs 10 gelöst. Weitere Ausgestaltungen der Erfindung ergeben sich aus den jeweiligen Unteransprüchen.

### Erfindungsgemäßes Verfahren:

Verfahren zur Wiedergewinnung von Methanol aus Prozessabgasen der Methanolsynthese, umfassend folgende Verfahrensschritte:
(a) Umsetzen eines Synthesegasstroms unter Methanolsynthesebedingungen in mindestens einem Methanolsynthesereaktor, der in einem Synthesegaskreislauf für nicht umgesetztes Synthesegas angeordnet ist,
(b) Ausleiten eines flüssigen Rohmethanolstroms aus dem Synthesegaskreislauf und Ausleiten eines Purgegasstroms aus dem Synthesegaskreislauf, wobei der Purgegasstrom mit Methanoldampf beladen ist,
(c) Einleiten des Purgegasstroms in eine Purgegaswaschvorrichtung, Inkontaktbringen des Purgegasstroms in der Purgegaswaschvorrichtung mit einem Wasserstrom als Waschmittel, das im Gegenstrom zu dem Purgegasstrom durch die Purgegaswaschvorrichtung geführt wird, wobei der Wasserstrom mindestens einen Teil des Methanols aus dem Purgegasstrom aufnimmt,
(d) Ausleiten eines mit Methanol beladenen Wasserstroms und eines an Methanol abgereicherten Purgegasstroms aus der Purgegaswaschvorrichtung,
(e) Einleiten des flüssigen Rohmethanolstroms in einen Entspannungsbehälter, in dem der Druck des flüssigen Rohmethanolstroms reduziert wird, wobei ein mit Methanol beladener Entspannungsgasstrom und ein entspannter Rohmethanolstrom erhalten werden,
(f) Einleiten des Entspannungsgasstroms in eine Entspannungsgaswaschvorrichtung, Inkontaktbringen des Entspannungsgasstroms in der Entspannungsgaswaschvorrichtung mit einem Wasserstrom als Waschmittel, das im Gegenstrom zu dem Entspannungsgasstrom durch die Entspannungsgaswaschvorrichtung geführt wird, wobei der Wasserstrom mindestens einen Teil des Methanols aus dem Entspannungsgasstrom aufnimmt,
(g) Ausleiten eines mit Methanol beladenen Wasserstroms und eines an Methanol abgereicherten Entspannungsgasstroms aus der Entspannungsgaswaschvorrichtung, wobei der mit Methanol beladene Wasserstrom dem entspannten Rohmethanolstrom zugegeben wird,
(h) Einleiten des entspannten Rohmethanolstroms in einen Lagertank, in dem es bis zu seiner Weiterverarbeitung oder Weiterverwendung gelagert und dabei von einem Inertisierungsgas überdeckt wird, das dabei mit Methanol beladen wird,
(i) Ausleiten eines mit Methanol beladenen Inertisierungsgasstroms aus dem Lagertank und Einleiten in eine Inertisierungsgaswaschvorrichtung, Inkontaktbringen des Inertisierungsgasstroms in der Inertisierungsgaswaschvorrichtung mit einem Wasserstrom als Waschmittel, das im Gegenstrom zu dem Inertisierungsgasstrom durch die Inertisierungsgaswaschvorrichtung geführt wird, wobei der Wasserstrom mindestens einen Teil des Methanols aus dem Inertisierungsgasstrom aufnimmt,
(j) Ausleiten eines mit Methanol beladenen Wasserstroms und eines an Methanol abgereicherten Inertisierungsgasstroms aus der Inertisierungsgaswaschvorrichtung.

### Erfindungsgemäße Vorrichtung:

Vorrichtung zur Wiedergewinnung von Methanol aus Prozessabgasen der Methanolsynthese, umfassend folgende Bestandteile und Baugruppen:
(a) einen Methanolsynthesereaktor, der in einem Synthesegaskreislauf für nicht umgesetztes Synthesegas angeordnet ist,
(b) eine Leitung zum Ausleiten eines flüssigen Rohmethanolstroms aus dem Synthesegaskreislauf und eine Leitung zum Ausleiten eines Purgegasstroms aus dem Synthesegaskreislauf, wobei der Purgegasstrom mit Methanoldampf beladen ist,
(c) eine als Gegenstromwäscher ausgestaltete Purgegaswaschvorrichtung, eine Leitung zum Einleiten des Purgegasstroms in eine Purgegaswaschvorrichtung, eine Leitung zum Zuführen eines Wasserstroms als Waschmittel, eine Leitung zum Ausleiten eines mit Methanol beladenen Wasserstroms und eine Leitung zum Ausleiten eines an Methanol abgereicherten Purgegasstroms aus der Purgegaswaschvorrichtung,
(d) einen Entspannungsbehälter, in dem der Druck des flüssigen Rohmethanolstroms reduziert wird, eine Leitung zum Einleiten des flüssigen Rohmethanolstroms in den Entspannungsbehälter, eine mit dem Entspannungsbehälter verbundene, als Gegenstromwäscher ausgestaltete Entspannungsgaswaschvorrichtung, eine Leitung zum Einleiten eines Wasserstroms als Waschmittel in die Entspannungsgaswaschvorrichtung, eine Leitung zum Ausleiten eines entspannten Rohmethanolstroms und eine Leitung zum Ausleiten eines an Methanol abgereicherten Entspannungsgasstroms,
(e) einen Lagertank, eine Leitung zum Einleiten des entspannten Rohmethanolstroms in den Lagertank, eine Leitung zum Einleiten eines Inertisierungsgases und eine Leitung zum Ausleiten eines mit Methanol beladenen Inertisierungsgasstroms aus dem Lagertank, eine Leitung zum Ausleiten des Rohmethanols,
(f) eine als Gegenstromwäscher ausgestaltete Inertisierungsgaswaschvorrichtung, eine Leitung zum Einleiten eines mit Methanol beladenen Inertisierungsgasstroms in die Inertisierungsgaswaschvorrichtung, eine Leitung zum Einleiten eines Wasserstroms als Waschmittel in die Inertisierungsgaswaschvorrichtung, eine Leitung zum Ausleiten eines an Methanol abgereicherten Inertisierungsgasstroms und eine Leitung zum Ausleiten eines mit Methanol beladenen Wasserstroms aus der Inertisierungsgaswaschvorrichtung.

Die für die Umsetzung von Synthesegas zu Methanol erforderlichen Methanolsynthesebedingungen sind dem Fachmann aus dem Stand der Technik, beispielsweise den eingangs erörterten Druckschriften, bekannt. Dies betrifft insbesondere, aber nicht ausschließlich, Verfahrensbedingungen wie Temperaturen, Drücke, geeignete Raumgeschwindigkeiten und zu verwendende Katalysatoren. Notwendige Anpassungen dieser Bedingungen an die jeweiligen Betriebserfordemisse wird er auf der Grundlage von Routineversuchen vornehmen.

Unter Purgegas oder alternativ Spülgas wird ein aus einem System, beispielsweise aus dem Synthesegaskreislauf der Methanolsynthese, entnommener Gasanteil verstanden, der zumeist gegenüber dem im System vorhandenen Gasinventar klein ist. Die Entnahme des Purgegases verhindert das stetige Anwachsen des Gasinventars des Systems und dient der Ausschleusung von Verunreinigungen, Nebenprodukten und/oder Inertkomponenten.

Unter Rohmethanol wird das direkt der Methanolsynthese entstammende Primärprodukt vor der destillativen Aufarbeitung zu Reinmethanol verstanden. Jedoch können bereits erste Aufarbeitungs- oder Konditionierungsschritte am Rohmethanol durchgeführt werden.

Das als Waschmittel verwendete Wasser ist zumeist demineralisiertes Wasser. Es können aber auch andere, insbesondere Wasserqualitäten höherer Reinheit, beispielsweise Reinstwasser oder destilliertes Wasser, als Waschmittel eingesetzt werden. Wasser geringerer Reinheit ist dann als Waschmittel einsetzbar, wenn die vorhandenen Begleitstoffe in nachgeschalteten Verfahrensstufen keine Probleme bereiten und die Produktspezifikationen des Reinmethanol-Produkts eingehalten werden.

### Bevorzugte Ausgestaltungen der Erfindung

In bevorzugter Ausgestaltung des erfindungsgemäßen Verfahrens wird der in Schritt (d) erhaltene, an Methanol abgereicherte Purgegasstrom einer Wasserstoffwiedergewinnungsvorrichtung zugeführt. Als Wasserstoffwiedergewinnungsvorrichtung kann dabei beispielsweise eine Druckwechseladsorptionsanlage (pressure swing adsorption, PSA) oder eine Membrananlage dienen. Auf diese Weise kann wertvoller Wasserstoff aus dem Purgegasstrom zurückgewonnen und zur Methanolsynthese zurückgeführt werden.

In einem weiteren Aspekt des erfindungsgemäßen Verfahrens wird der in Schritt (d) erhaltene, mit Methanol beladene Wasserstrom mindestens teilweise dem Entspannungsbehälter zugeführt. Auf diese Weise kann der mit Methanol beladene Wasserstrom gemeinsam mit anderen Methanol enthaltenden Wasserströmen der weiteren Aufarbeitung zugeführt werden, so dass keine separate Aufarbeitungsvorrichtungen für den in Schritt (d) erhaltenen, mit Methanol beladenen Wasserstrom benötigt werden.

In weiterer bevorzugter Ausgestaltung des erfindungsgemäßen Verfahrens wird der in Schritt (d) erhaltene, mit Methanol beladene Wasserstrom mindestens teilweise als Waschmittel in der Entspannungsgaswaschvorrichtung verwendet. Vorteilhaft ist es dabei, dass das zumeist als Waschmittel verwendete, demineralisierte Wasser zum Teil oder vollständig eingespart wird, wodurch die Kosten für seine Bereitstellung reduziert werden.

Vorteilhaft ist es ferner, wenn mindestens ein Teil des in Verfahrensschritt (e) erhaltenen, entspannten Rohmethanolstroms ohne Zwischenlagerung im Lagertank einer Destillationsvorrichtung zugeführt wird. Hierdurch können Lagerkapazitäten für Rohmethanol eingespart und die entsprechenden Lagertanks kleiner ausgelegt werden.

Es ist in besonderer Ausgestaltung der Erfindung möglich, mindestens einen Teil des in Verfahrensschritt (g) erhaltenen, an Methanol abgereicherten Entspannungsgasstroms als Inertisierungsgas in den Lagertank einzuleiten. Diese Ausgestaltung der Erfindung ermöglicht es, Inertisierungsgase wie beispielsweise Stickstoff ganz oder teilweise einzusparen.

Günstig ist es zudem, wenn mindestens ein Teil des in Verfahrensschritt (j) erhaltenen, mit Methanol beladenen Wasserstroms als Waschmittel in der Entspannungsgaswaschvorrichtung verwendet wird. Vorteilhaft ist es dabei, dass das zumeist als Waschmittel verwendete, demineralisierte Wasser zum Teil oder vollständig eingespart wird, wodurch die Kosten für seine Bereitstellung reduziert werden.

Weiterhin hat es sich als vorteilhaft herausgestellt, wenn mindestens ein Teil des in Verfahrensschritt (j) erhaltenen, mit Methanol beladenen Wasserstroms dem Entspannungsbehälter zugeführt wird. Auf diese Weise kann der mit Methanol beladene Wasserstrom gemeinsam mit anderen Methanol enthaltenden Wasserströmen der weiteren Aufarbeitung zugeführt werden, so dass keine separate Aufarbeitungsvorrichtungen für den in Schritt (d) erhaltene, mit Methanol beladene Wasserstrom benötigt werden.

Besonders bevorzugt wird das erfindungsgemäße Verfahren zur Wiedergewinnung von Methanol aus Prozessabgasen einer Anlage zur Methanolsynthese verwendet, in der Methanol aus kohlebasiertern Synthesegas hergestellt wird. Kohlebasiertes Synthesegas zeichnet sich aufgrund des C/H-Verhältnisses im Einsatzstoff Kohle insbesondere durch einen hohen Gehalt an Kohlenmonoxid, aber einen relativ geringen Wassergehalt aus. Ein definierter, gegenüber dem ursprünglichen Gehalt im kohlebasierten Synthesegas erhöhter Wassergehalt ist aber durchaus erwünscht. Er bietet bei der destillativen Aufarbeitung des Rohmethanols Vorteile, da auf diese Weise unerwünschte unpolare Verunreinigungen, beispielsweise als Nebenprodukte der Methanolsynthese entstandene Alkane, leichter aus dem Methanol abgetrennt werden können. Daher ist die Beaufschlagung des Rohmethanols mit Wasser als Waschmittel an verschiedenen Stellen des Verfahrens durchaus vorteilhaft.

In besonderer Ausgestaltung der erfindungsgemäßen Vorrichtung umfasst diese ferner eine Wasserstoffwiedergewinnungsvorrichtung und eine Leitung zum Einleiten eines an Methanol abgereicherten Purgegasstroms in die Wasserstoffwiedergewinnungsvorrichtung. Als Wasserstoffwiedergewinnungsvorrichtung kann dabei beispielsweise eine Druckwechseladsorptionsanlage (pressure swing adsorption, PSA) oder eine Membrananlage dienen. Auf diese Weise kann wertvoller Wasserstoff aus dem Purgegasstrom zurückgewonnen und zur Methanolsynthese zurückgeführt werden.

Bevorzugt umfasst die erfindungsgemäße Vorrichtung ferner eine Leitung zum Zuführen des in Verfahrensschritt (d) erhaltenen, mit Methanol beladenen Wasserstroms zum Entspannungsbehälter. Auf diese Weise kann der mit Methanol beladene Wasserstrom gemeinsam mit anderen Methanol enthaltenden Wasserströmen der weiteren Aufarbeitung zugeführt werden, so dass keine separate Aufarbeitungsvorrichtungen für den in Schritt (d) erhaltene, mit Methanol beladene Wasserstrom benötigt werden.

In einem weiteren Aspekt umfasst die erfindungsgemäße Vorrichtung ferner eine Leitung zum Zuführen des in Verfahrensschritt (d) erhaltenen, mit Methanol beladenen Wasserstroms als Waschmittel zu der Entspannungsgaswaschvorrichtung. Vorteilhaft ist es dabei, dass das zumeist als Waschmittel verwendete, demineralisierte Wasser zum Teil oder vollständig eingespart wird, wodurch die Kosten für seine Bereitstellung reduziert werden.

Günstigerweise umfasst die erfindungsgemäße Vorrichtung ferner eine Destillationsvorrichtung und eine Leitung zum Zuführen des in Verfahrensschritt (e) erhaltenen, entspannten Rohmethanolstroms zu der Destillationsvorrichtung. Hierdurch können Lagerkapazitäten für Rohmethanol eingespart und die entsprechenden Lagertanks kleiner ausgelegt werden.

In einem weiteren Aspekt umfasst die erfindungsgemäße Vorrichtung ferner eine Leitung zum Zuführen mindestens eines Teils des in Verfahrensschritt (g) erhaltenen, an Methanol abgereicherten Entspannungsgasstroms als Inertisierungsgas in den Lagertank. Diese Ausgestaltung der Erfindung ermöglicht es, Inertisierungsgase wie beispielsweise Stickstoff ganz oder teilweise einzusparen.

Günstig ist es, wenn die erfindungsgemäße Vorrichtung ferner eine Leitung zum Zuführen mindestens eines Teils des in Verfahrensschritt (j) erhaltenen, mit Methanol beladenen Wasserstroms als Waschmittel zu der Entspannungsgaswaschvorrichtung umfasst. Vorteilhaft ist es dabei, dass das zumeist als Waschmittel verwendete, demineralisierte Wasser zum Teil oder vollständig eingespart wird, wodurch die Kosten für seine Bereitstellung reduziert werden.

Bevorzugt umfasst die erfindungsgemäße Vorrichtung ferner Leitung zum Zuführen mindestens eines Teils des in Verfahrensschritt (j) erhaltenen, mit Methanol beladenen Wasserstroms zum Entspannungsbehälter. Auf diese Weise kann der mit Methanol beladene Wasserstrom gemeinsam mit anderen Methanol enthaltenden Wasserströmen der weiteren Aufarbeitung zugeführt werden, so dass keine separate Aufarbeitungsvorrichtungen für den in Schritt (d) erhaltene, mit Methanol beladene Wasserstrom benötigt werden.

Besonders bevorzugt befindet sich die erfindungsgemäße Vorrichtung in einer Anlage zur Herstellung von Methanol durch Umsetzung kohlebasierten Synthesegases. Kohlebasiertes Synthesegas zeichnet sich aufgrund des C/H-Verhältnisses im Einsatzstoff Kohle insbesondere durch einen hohen Gehalt an Kohlenmonoxid, aber einen relativ geringen Wassergehalt aus. Ein definierter, gegenüber dem ursprünglichen Gehalt im kohlebasierten Synthesegas erhöhter Wassergehalt ist aber durchaus erwünscht. Er bietet bei der destillativen Aufarbeitung des Rohmethanols Vorteile, da auf diese Weise unerwünschte unpolare Verunreinigungen, beispielsweise als Nebenprodukte der Methanolsynthese entstandene Alkane, leichter aus dem Methanol abgetrennt werden können. Daher ist die Beaufschlagung des Rohmethanols mit Wasser als Waschmittel an verschiedenen Stellen des Verfahrens durchaus vorteilhaft.

### Ausführungs- und Zahlenbeispiele

Weitere Merkmale, Vorteile und Anwendungsmöglichkeiten der Erfindung ergeben sich auch aus der nachfolgenden Beschreibung von Ausführungs- und Zahlenbeispielen und der Zeichnungen. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung.

Es zeigen:
- Fig. 1: die schematische Darstellung des erfindungsgemäßen Verfahrens bzw. der erfindungsgemäßen Vorrichtung nach einer ersten Ausgestaltung,
- Fig. 2: die schematische Darstellung des erfindungsgemäßen Verfahrens bzw. der erfindungsgemäßen Vorrichtung nach einer zweiten Ausgestaltung,
- Fig. 3: die schematische Darstellung des erfindungsgemäßen Verfahrens bzw. der erfindungsgemäßen Vorrichtung nach einer dritten Ausgestaltung,
- Fig. 4: die schematische Darstellung des erfindungsgemäßen Verfahrens bzw. der erfindungsgemäßen Vorrichtung nach einer vierten Ausgestaltung.

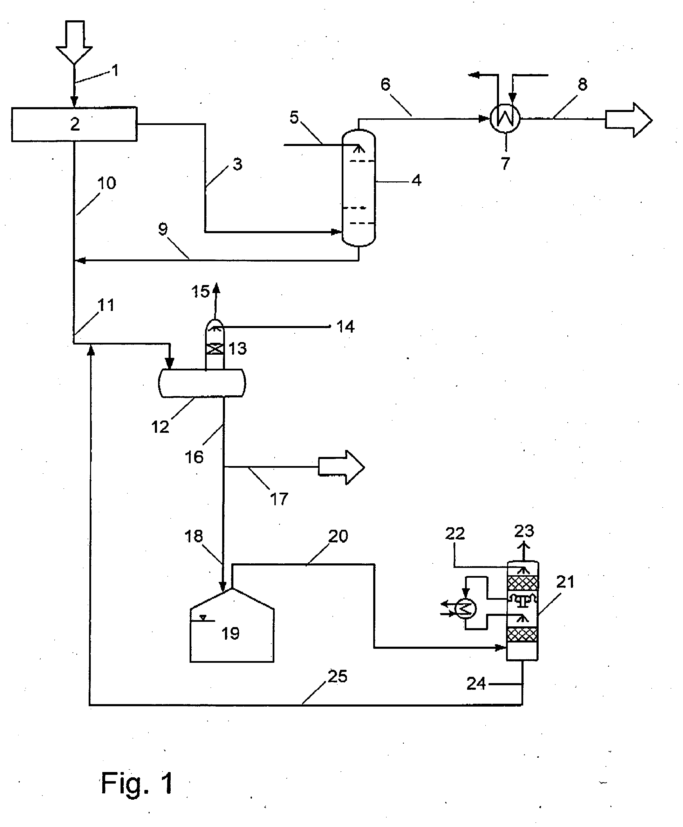

In Fig. 1 wird das erfindungsgemäße Verfahren bzw. die erfindungsgemäße Vorrichtung nach einer ersten Ausgestaltung erläutert. Über die Leitung 1 wird aus Wasserstoff und Kohlenoxiden bestehendes Synthesegas in den hier nur schematisch dargestellten und nicht weiter im Detail erläuterten, in einem Synthesegaskreislauf angeordneten Methanolsynthesereaktor 2 eingeleitet, in dem das Synthesegas unter Bedingungen der Methanolsynthese teilweise zu Methanol umgesetzt wird. Das dabei erzeugte Rohmethanol wird über Leitung 10 aus dem Methanolsynthesereaktor ausgeleitet.

Der größte Teil des bei der Methanolsynthese nicht umgesetzten Synthesegases wird über den bildlich nicht dargestellten Synthesegaskreislauf zum Eingang des Methanolsynthesereaktors zurückgeführt. Der verbliebene Anteil des nicht umgesetzten Synthesegases wird als Purgegas oder Spülgas über Leitung 3 aus dem Methanolsynthesereaktor ausgeleitet und zu der Purgegaswaschvorrichtung 4 geführt. Es handelt sich dabei um eine an sich bekannte Waschkolonne, die mit Böden, Füllkörperschüttungen oder strukturierten Packungen ausgerüstet sein kann, um den Stoffaustausch zwischen Gas und Flüssigkeit zu intensivieren. Der Purgegaswaschvorrichtung wird der Purgegasstrom an deren Unterseite aufgegeben und im Gegenstrom in Kontakt mit einem über Leitung 5 als Waschmittel herangeführten Wasserstrom gebracht, wodurch sein Gehalt an Methanoldampf reduziert wird. Im vorliegenden und in den nachfolgend beschriebenen Ausführungsbeispielen wird - sofern nicht anders vermerkt - demineralisiertes Wasser als Waschmittel verwendet.

Das an Methanol abgereicherte Purgegas verlässt über Leitung 6 die Purgegaswaschvorrichtung. Es kann dann optional in Wärmetauscher 7 im indirekten Wärmetausch gegen Niederdruckdampf als Heizmedium erhitzt und über Leitung 8 aus dem Verfahren ausgeleitet und zu einer bildlich nicht dargestellten Wasserstoffrückgewinnungsanlage geführt werden.

Das in der Purgegaswaschvorrichtung mit Methanol beladene Wasser wird über Leitung 9 aus dieser ausgeleitet und gemeinsam mit dem über Leitung 10 herangeführten Rohmethanol über Leitung 11 zu dem Entspannungsbehälter 12 geführt. In diesem wird das Rohmethanol-Wasser-Gemisch von 7,0 MPa,g auf einen Druck von 0,5 MPa,g entspannt (,g bezeichnet die entsprechende Druckeinheit bei Überdruck). Die bei der Entspannung freiwerdenden Gase bzw. Dämpfe werden in der baulich mit dem Entspannungsbehälter verbundenen und mit diesem in Fluidverbindung stehenden Entspannungsgaswaschvorrichtung 13 in Kontakt mit einem über Leitung 14 als Waschmittel herangeführten Wasserstrom gebracht, wodurch sein Gehalt an Methanoldampf reduziert wird. Auch bei der Entspannungsgaswaschvorrichtung handelt es sich um eine an sich bekannte Waschkolonne, die mit Böden, Füllkörperschüttungen oder strukturierten Packungen ausgerüstet sein kann, um den Stoffaustausch zwischen Gas und Flüssigkeit zu intensivieren. Der an Methanol abgereicherte Entspannungsgasstrom verlässt über Leitung 15 die Entspannungsgaswaschvorrichtung.

Das entspannte Rohmethanol-Wasser-Gemisch wird über Leitung 16 aus dem Entspannungsbehälter 12 ausgeleitet. Über Leitung 17 kann es vollständig oder teilweise zu einer bildlich nicht dargestellten Destillationsvorrichtung geführt werden, in der das Rohmethanol durch destillative Wasserabtrennung zu Reinmethanol weiterverarbeitet wird.

Der nicht zu der Destillationsvorrichtung geführte Anteil des Rohmethanol-Wasser-Gemischs wird über Leitung 18 zum Tank 19 geführt und in diesen eingeleitet. Aus dem Tank kann zu einem späteren Zeitpunkt Rohmethanol entnommen und der destillativen Aufarbeitung zugeführt werden. Zuführ- und Entnahmeleitungen für Rohmethanol sind bildlich nicht dargestellt. Bei dem Tank 19 handelt es sich um einen Festdachtank, in dem das freie Innenvolumen des Tanks durch Stickstoff, der über eine nicht bildlich gezeigte Leitung herangeführt wird, ausgefüllt und somit inertisiert wird. Die Stickstoffatmosphäre sättigt sich dabei mit Methanol. Bei Beladung der Tanks oder durch Tankatmung, beispielsweise durch Sonneneinstrahlung, wird mit Methanol beladener Stickstoff aus dem Tank über Leitung 20 ausgeleitet, um einen Überdruck im Tank zu vermeiden. Dieser mit Methanoldampf beladene Inertisierungsgasstrom wird zu der Inertisierungsgaswaschvorrichtung 21 geführt, dieser an ihrer Unterseite aufgegeben und im Gegenstrom in Kontakt mit einem über Leitung 22 als Waschmittel herangeführten Wasserstrom gebracht, wodurch sein Gehalt an Methanoldampf reduziert wird.

Auch die Inertisierungsgaswaschvorrichtung ist als Waschkolonne ausgestaltet und mit Böden, Füllkörperschüttungen oder strukturierten Packungen ausgerüstet, um den Stoffaustausch zwischen Gas und Flüssigkeit zu intensivieren. Sie ist jedoch durch einen etwa in der Kolonnenmitte angeordneten Trennboden, der als Kaminboden ausgestaltet ist, in einen oberen und einen unteren Teil getrennt. Auf diesem sammelt sich das mit Methanol teilbeladene Waschmittel, da es durch die Ausgestaltung als Kaminboden nicht innerhalb der Kolonne in den unteren Kolonnenteil gelangen kann, wohingegen der zu reinigende Inertisierungsgasstrom frei von dem unteren in den oberen Kolonnenteil strömen kann. Das mit Methanol teilbeladene Waschmittel wird dagegen über eine Leitung aus der Kolonne herausgeführt, in einem Zwischenkühler gekühlt und der Kolonne am oberen Ende des unteren Kolonnenteils wieder aufgegeben. Durch die Abkühlung des teilbeladenen Waschmittels erhöht sich dessen Aufnahmefähigkeit für weiteres Methanol.

Über Leitung 24 wird das mit Methanol beladene Waschmittel aus der Inertisierungsgaswaschvorrichtung ausgeleitet und über die Leitungen 25 und 11 zu dem Entspannungsbehälter 12 geführt. Falls in den Leitungen 25 und 11 deutlich unterschiedliche Drücke herrschen, kann das mit Methanol beladene Waschmittel auch getrennt von der Leitung 11 zu dem Entspannungsbehätter geführt werden und/oder es wird bei der Zusammenführung von Leitung 25 und Leitung 11 ein Entspannungsventil vogesehen.

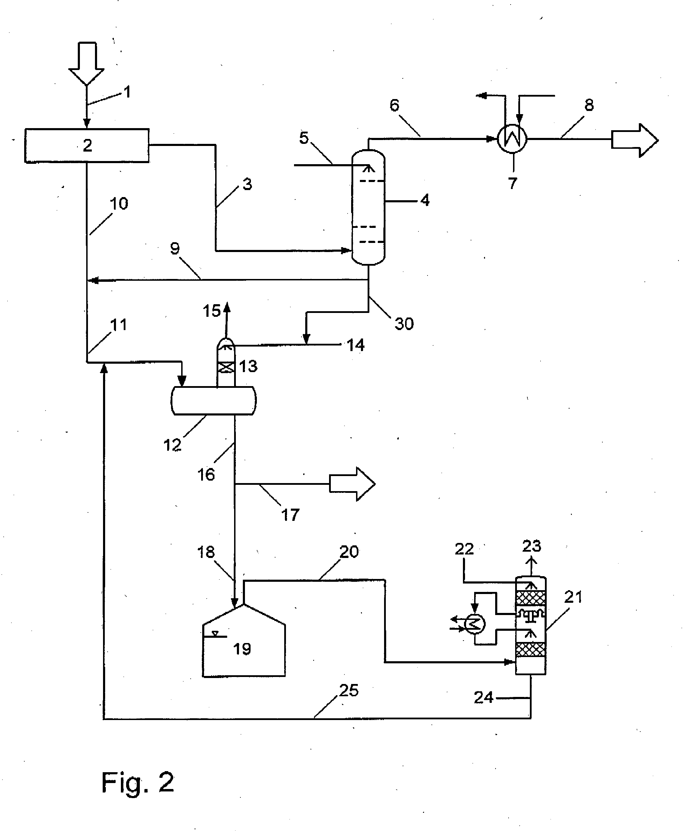

In Fig. 2 ist schematisch der Ablauf des erfindungsgemäßen Verfahrens bzw. der Aufbau einer erfindungsgemäßen Vorrichtung nach einer zweiten Ausgestaltung dargestellt. Das erfindungsgemäße Verfahren bzw. die erfindungsgemäße Vorrichtung entspricht bis zu der Anlagenkomponente 25 der in Fig. 1 dargestellten Ausgestaltung. Es tritt aber nun eine Leitung 30 hinzu, mit der der aus der Purgegaswaschvorrichtung ausgeleitete, mit Methanol beladene Wasserstrom mindestens teilweise der Entspannungsgaswaschvorrichtung und schließlich dem Entspannungsbehälter zugeführt wird. Auf diese Weise kann der mit Methanol beladene Wasserstrom gemeinsam mit anderen Methanol enthältenden Wasserströmen der weiteren Aufarbeitung zugeführt werden, so dass keine separate Aufarbeitungsvorrichtungen für den in der Purgegaswaschvorrichtung erhaltenen, mit Methanol beladene Wasserstrom benötigt werden. Zudem kann der mit Methanol beladene Wasserstrom mindestens einen Teil des als Waschmittel verwendeten, demineralisierten Wassers ersetzen.

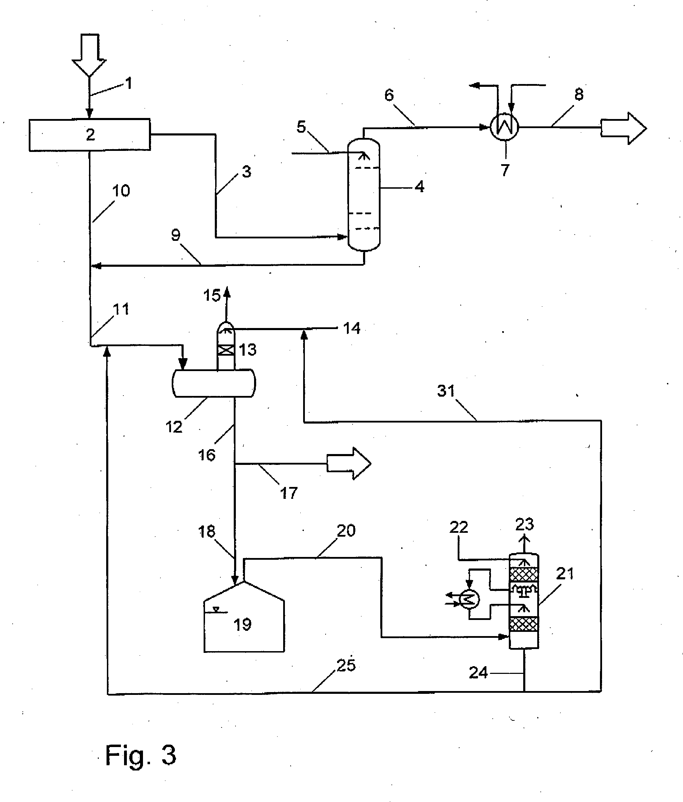

In Fig. 3 ist schematisch der Ablauf des erfindungsgemäßen Verfahrens bzw. der Aufbau einer erfindungsgemäßen Vorrichtung nach einer dritten Ausgestaltung dargestellt. Das erfindungsgemäße Verfahren bzw. die erfindungsgemäße Vorrichtung entspricht bis zu der Anlagenkomponente 25 der in Fig. 1 dargestellten Ausgestaltung. Es tritt aber nun eine Leitung 31 hinzu, mit der der aus der Inertisierungsgaswaschvorrichtung ausgeleitete, mit Methanol beladene Wasserstrom mindestens teilweise der Entspannungsgaswaschvorrichtung und schließlich dem Entspannungsbehälter zugeführt wird. Auf diese Weise kann der mit Methanol beladene Wasserstrom gemeinsam mit anderen Methanol enthaltenden Wasserströmen der weiteren Aufarbeitung zugeführt werden, so dass keine separate Aufarbeitungsvorrichtungen für den in der Inertisierungsgaswaschvorrichtung erhaltenen, mit Methanol beladene Wasserstrom benötigt werden. Zudem kann der mit Methanol beladene Wasserstrom mindestens einen Teil des als Waschmittel verwendeten, demineralisierten Wassers ersetzen.

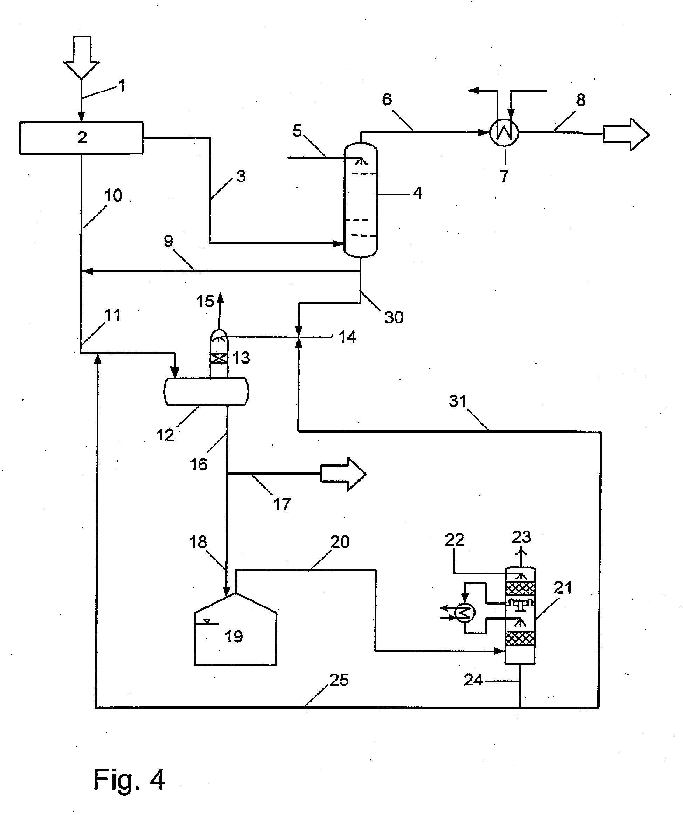

In Fig. 4 ist schematisch der Ablauf des erfindungsgemäßen Verfahrens bzw. der Aufbau einer erfindungsgemäßen Vorrichtung nach einer vierten Ausgestaltung dargestellt. Das erfindungsgemäße Verfahren bzw. die erfindungsgemäße Vorrichtung entspricht bis zu der Anlagenkomponente 25 der in Fig. 1 dargestellten Ausgestaltung. Es treten aber nun sowohl die Leitung 30 als auch die Leitung 31 hinzu, deren Funktion in den in Fig. 2 und Fig. 3 gezeigten Ausgestaltungen gezeigt und im Zusammenhang mit diesen oben erörtert wurde. Durch die in Fig. 4 gezeigte Ausgestaltung kann ein besonders großer Anteil des als Waschmittel verwendeten, demineralisierten Wassers durch mit Methanol beladene Wasserströme aus der Purgegaswaschvorrichtung und der Inertisierungsgaswaschvorrichtung ersetzt werden.

### Zahlenbeispiele

In den nachfolgenden Tabellen sind Zahlenbeispiele für die erfindungsgemäße Reinigung von Purgegas (Tabelle 1), von Expansionsgas (Tabelle 2) und von Tankabgas (Inertisierungsgas) (Tabelle 3) zusammengestellt. Ferner zeigen Tabellen 4 und 5 die Reinigung von Expansionsgas nach besonderen Ausgestaltungen der Erfindung, in denen teilbeladenes Abwasser aus der Purgegaswaschvorrichtung (Tabelle 4, Leitung 30) oder aus der Inertisierungsgaswaschvorrichtung (Tabelle 5, Leitung 31) zu der Entspannungsgaswaschvorrichtung zurückgeführt und dort als Waschmittel eingesetzt wird.

| **Tabelle 1: Purgegas-Reinigung** | | | | |
|---|---|---|---|---|
| | Purgegas | Demin. Wasser | Purgegas gereinigt | Abwasser |
| Molenbruch | | | | |
| CH3OH | 0,057 | 0,0000 | 0,0000 | 0,0461 |
| H2O | 0,0001 | 1,0000 | 0,0015 | 0,9515 |
| CO2 | 0,0424 | 0,0000 | 0,0424 | 0,0013 |
| CO | 0,0643 | 0.0000 | 0,0646 | 0,0001 |
| H2 | 0,7990 | 0,0000 | 0,8026 | 0,0009 |
| AR | 0.0000 | 0,0000 | 0,0000 | 0,9000 |
| N2 | 0,0883 | 0,0000 | 0,0887 | 0,0001 |
| CH4 | 0,0002 | 0,0000 | 0,0002 | 0.0000 |
| Gesamtstrom kmol/hr | 1288,1 | 152,9 | 1282,1 | 158,9 |
| Gesamtstrom kg/hr | 10223.7 | 2754.9 | 10010,3 | 2968,3 |
| Temperatur °C | 40,0 | 42,2 | 43,9 | 44,7 |
| Druck MPag | 7,0 | 8.1 | 7.0 | 7.0 |

| **Tabelle 2: Entspannungsgas-Reinigung** | | | |
|---|---|---|---|
| | Entspannungsgas | Demin. Wasser | Entspannungsgas gereinigt |
| Molenbruch | | | |
| CH3OH | 0,0579 | 0,0000 | 0.0000 |
| H2O | 0,0018 | 1,0000 | 0,0132 |
| CO2 | 0,2285 | 0,0000 | 0,2392 |
| CO | 0,0397 | 0,0000 | 0,0417 |
| H2 | 0.3607 | 0,0000 | 0,3789 |
| AR | 0,0379 | 0,0000 | 0,0398 |
| N2 | 0,2676 | 0,0000 | 0,2812 |
| CH4 | 0,0058 | 0,0000 | 0,0061 |
| Gesamtstrom kmol/hr | 177.9 | 277,5 | 169,3 |
| Gesamtstrom kg/hr | 4072,5 | 5000,0 | 3769,8 |
| Temperatur °C | 40,6 | 40,0 | 40,4 |
| Druck MPag | 0.5 | 0.5 | 0.5 |

| **Tabelle 3: Inertisierungsgas-Relnigung** | | | | |
|---|---|---|---|---|
| | Inertisierungsgas | Demin. Wasser | Inertisierungsgas gereinigt | Abwasser |
| Molenbruch | | | | |
| CH3OH | 0,3338 | 0,0000 | 0,0043 | 0,2671 |
| H2O | 0,0000 | 1.0000 | 0,1425 | 0,7328 |
| CO2 | 0,0000 | 0,0000 | 0,0000 | 0,0000 |
| CO | 0,0000 | 0,0000 | 0.0000 | 0,0000 |
| H2 | 0,0000 | 0,0000 | 0.0000 | 0,0000 |
| AR | 0.0000 | 0.0000 | 0,0000 | 0.0000 |
| N2 | 0,6662 | 0.0000 | 0,8532 | 0.0000 |
| CH4 | 0,0000 | 0,0000 | 0,0000 | 0,0000 |
| Gesamtstrom kmol/hr | 180,0 | 183,2 | 140,5 | 222,6 |
| Gesamtstrom kg/hr | 5284,5 | 3300,0 | 3739,2 | 4845,3 |
| Temperatur °C | 40,3 | 40,0 | 53,1 | 50,2 |
| Druck MPag | 0,0 | 0,4 | 0,0 | 0,5 |

| **Tabelle 4: Entspannungsgas-Reinigung mit Leitung (30)** | | | |
|---|---|---|---|
| | Entspannungsgas | Abwasser | Entspannungsgas gereinigt |
| Molenbruch | | | |
| CH3OH | 0.0575 | 0,0461 | 0,0082 |
| H2O | 0,0012 | 0,9515 | 0,0202 |
| CO2 | 0,2264 | 0,0013 | 0,2339 |
| CO | 0,0399 | 0,0001 | 0,0412 |
| H2 | 0,3625 | 0,0009 | 0,3745 |
| AR | 0,0380 | 0.0000 | 0,0392 |
| N2 | 0,2686 | 0.0001 | 0,2769 |
| CH4 | 0,0058 | 0.0000 | 0,0060 |
| Gesamtstrom kmol/hr | 176,9 | 158,9 | 171,7 |
| Gesamtstrom kg/hr | 4036,8 | 2968,3 | 3819,4 |
| Temperatur °C | 39,9 | 44,6 | 49,6 |
| Druck MPag | 0,5 | 0,5 | 0,5 |

| **Tabelle 5: Entspannungsgas-Reinigung mit Leitung (31)** | | | |
|---|---|---|---|
| | Entspannungsgas | Abwasser | Entspannungsgas gereinigt |
| Molenbruch | | | |
| CH3OH | 0,0571 | 0,2671 | 0,0344 |
| H2O | 0,0012 | 0,7328 | 0,0165 |
| CO2 | 0,2259 | 0.0000 | 0,2263 |
| CO | 0,0399 | 0,0000 | 0,0403 |
| H2 | 0,3630 | 0.0000 | 0,3666 |
| AR | 0,0380 | 0,0000 | 0,0384 |
| N2 | 0,2690 | 0.0000 | 0,2716 |
| CH4 | 0,0058 | 0.0000 | 0,0058 |
| Gesamtstrom kmol/hr | 176,7 | 222,6 | 174,9 |
| Gesamtstrom kg/hr | 4027,1 | 4845,3 | 3929,7 |
| Temperatur °C | 39,7 | 50,0 | 49,7 |
| Druck MPag | 0,5 | 0,5 | 0.5 |

### Gewerbliche Anwendbarkeit

Mit der Erfindung wird eine effiziente Wiedergewinnung von Methanol aus mit Methanol beladenen Abgasen in einem integrierten Fließschema zu Herstellung und Aufarbeitung von Methanol vorgeschlagen. Die dabei aus den Abgasen abgeschiedenen Methanolanteile werden innerhalb der bereits bestehenden, destillativen Aufarbeitung des Rohmethanols zu Reinmethanol zurückgewonnen, so dass keine gesonderten Vorrichtungen zur Rückgewinnung des Methanols aus den beladenen Wäscherabwässern benötigt werden. Der Wertstoff Methanol wird zurückgewonnen und die Belastung der Umwelt reduziert. Durch besondere Ausgestaltungen der Erfindung kann der Bedarf an Wasser als Waschmittel weiter reduziert werden.

### Bezugszeichenliste

- 1: Leitung
- 2: Methanolsynthesereaktor
- 3: Leitung
- 4: Purgegaswaschvorrichtung
- 5: Leitung
- 6: Leitung
- 7: Wärmetauscher
- 8: Leitung
- 9: Leitung
- 10: Leitung
- 11: Leitung
- 12: Entspannungsbehälter
- 13: Entspannungsgaswaschvorrichtung
- 14: Leitung
- 15: Leitung
- 16: Leitung
- 17: Leitung
- 18: Leitung
- 19: Tank
- 20: Leitung
- 21: Inertisierungsgaswaschvorrichtung
- 22: Leitung
- 23: Leitung
- 24: Leitung
- 25: Leitung
- 30: Leitung
- 31: Leitung

## Patentansprüche

1. Verfahren zur Wiedergewinnung von Methanol aus Prozessabgasen der Methanolsynthese, umfassend folgende Verfahrensschritte:
(a) Umsetzen eines Synthesegasstroms unter Methanolsynthesebedingungen in mindestens einem Methanolsynthesereaktor, der in einem Synthesegaskreislauf für nicht umgesetztes Synthesegas angeordnet ist,
(b) Ausleiten eines flüssigen Rohmethanolstroms aus dem Synthesegaskreislauf und Ausleiten eines Purgegasstroms aus dem Synthesegaskreislauf, wobei der Purgegasstrom mit Methanoldampf beladen ist,
(c) Einleiten des Purgegasstroms in eine Purgegaswaschvorrichtung, Inkontaktbringen des Purgegasstroms in der Purgegaswaschvorrichtung mit einem Wasserstrom als Waschmittel, das im Gegenstrom zu dem Purgegasstrom durch die Purgegaswaschvorrichtung geführt wird, wobei der Wasserstrom mindestens einen Teil des Methanols aus dem Purgegasstrom aufnimmt,
(d) Ausleiten eines mit Methanol beladenen Wasserstroms und eines an Methanol abgereicherten Purgegasstroms aus der Purgegaswaschvorrichtung,
(e) Einleiten des flüssigen Rohmethanolstroms in einen Entspannungsbehälter, in dem der Druck des flüssigen Rohmethanolstroms reduziert wird, wobei ein mit Methanol beladener Entspannungsgasstrom und ein entspannter Rohmethanolstrom erhalten werden,
(f) Einleiten des Entspannungsgasstroms in eine Entspannungsgaswaschvorrichtung, Inkontaktbringen des Entspannungsgasstroms in der Entspannungsgaswaschvorrichtung mit einem Wasserstrom als Waschmittel, das im Gegenstrom zu dem Entspannungsgasstrom durch die Entspannungsgaswaschvorrichtung geführt wird, wobei der Wasserstrom mindestens einen Teil des Methanols aus dem Entspannungsgasstrom aufnimmt,
(g) Ausleiten eines mit Methanol beladenen Wasserstroms und eines an Methanol abgereicherten Entspannungsgasstroms aus der Entspannungsgaswaschvorrichtung, wobei der mit Methanol beladene Wasserstrom dem entspannten Rohmethanolstrom zugegeben wird,
(h) Einleiten des entspannten Rohmethanolstroms in einen Lagertank, in dem es bis zu seiner Weiterverarbeitung oder Weiterverwendung gelagert und dabei von einem Inertisierungsgas überdeckt wird, das dabei mit Methanol beladen wird,
(i) Ausleiten eines mit Methanol beladenen Inertisierungsgasstroms aus dem Lagertank und Einleiten in eine Inertisierungsgaswaschvorrichtung, Inkontaktbringen des Inertisierungsgasstroms in der Inertisierungsgaswaschvorrichtung mit einem Wasserstrom als Waschmittel, das im Gegenstrom zu dem Inertisierungsgasstrom durch die Inertisierungsgaswaschvorrichtung geführt wird, wobei der Wasserstrom mindestens einen Teil des Methanols aus dem Inertisierungsgasstrom aufnimmt,
(j) Ausleiten eines mit Methanol beladenen Wasserstroms und eines an Methanol abgereicherten Inertisierungsgasstroms aus der Inertisierungsgaswaschvorrichtung.

2. Verfahren nach Anspruch 1, **dadurch gekennzeichnet, dass** der in Schritt (d) erhaltene, an Methanol abgereicherte Purgegasstrom einer Wasserstoffwiedergewinnungsvorrichtung zugeführt wird.

3. Verfahren nach Anspruch 1, **dadurch gekennzeichnet, dass** der in Schritt (d) erhaltene, mit Methanol beladene Wasserstrom mindestens teilweise dem Entspannungsbehälter zugeführt wird.

4. Verfahren nach Anspruch 1, **dadurch gekennzeichnet, dass** der in Schritt (d) erhaltene, mit Methanol beladene Wasserstrom mindestens teilweise als Waschmittel in der Entspannungsgaswaschvorrichtung verwendet wird.

5. Verfahren nach Anspruch 1, **dadurch, gekennzeichnet, dass** mindestens ein Teil des in Schritt (e) erhaltenen, entspannten Rohmethanolstroms ohne Zwischenlagerung im Lagertank einer Destillationsvorrichtung zugeführt wird.

6. Verfahren nach Anspruch 1, **dadurch gekennzeichnet, dass** mindestens ein Teil des in Schritt (g) erhaltenen, an Methanol abgereicherten Entspannungsgasstroms als Inertisierungsgas in den Lagertank eingeleitet wird.

7. Verfahren nach Anspruch 1, **dadurch gekennzeichnet, dass** mindestens ein Teil des in Schritt (j) erhaltenen, mit Methanol beladenen Wasserstroms als Waschmittel in der Entspannungsgaswaschvorrichtung verwendet wird.

8. Verfahren nach Anspruch 1, **dadurch gekennzeichnet, dass** mindestens ein Teil des in Schritt (j) erhaltenen, mit Methanol beladenen Wasserstroms dem Entspannungsbehälter zugeführt wird.

9. Verfahren nach einem der vorgenannten Ansprüche, **dadurch gekennzeichnet, dass** es zur Wiedergewinnung von Methanol aus Prozessabgasen einer Anlage zur Methanolsynthese verwendet wird, in der Methanol aus kohlebasiertem Synthesegas hergestellt wird.

10. Vorrichtung zur Wiedergewinnung von Methanol aus Prozessabgasen der Methanolsynthese, umfassend folgende Bestandteile und Baugruppen:
(a) einen Methanolsynthesereaktor, der in einem Synthesegaskreislauf für nicht umgesetztes Synthesegas angeordnet ist,
(b) eine Leitung zum Ausleiten eines flüssigen Rohmethanolstroms aus dem Synthesegaskreislauf und eine Leitung zum Ausleiten eines Purgegasstroms aus dem Synthesegaskreislauf, wobei der Purgegasstrom mit Methanoldampf beladen ist,
(c) eine als Gegenstromwäscher ausgestaltete Purgegaswaschvorrichtung, eine Leitung zum Einleiten des Purgegasstroms in eine Purgegaswaschvorrichtung, eine Leitung zum Zuführen eines Wasserstroms als Waschmittel, eine Leitung zum Ausleiten eines mit Methanol beladenen Wasserstroms und eine Leitung zum Ausleiten eines an Methanol abgereicherten Purgegasstroms aus der Purgegaswaschvorrichtung,
(d) einen Entspannungsbehälter, in dem der Druck des flüssigen Rohmethanolstroms reduziert wird, eine Leitung zum Einleiten des flüssigen Rohmethanolstroms in den Entspannungsbehälter, eine mit dem Entspannungsbehälter verbundene, als Gegenstromwäscher ausgestaltete Entspannungsgaswaschvorrichtung, eine Leitung zum Einleiten eines Wasserstroms als Waschmittel in die Entspannungsgaswaschvorrichtung, eine Leitung zum Ausleiten eines entspannten Rohmethanolstroms und eine Leitung zum Ausleiten eines an Methanol abgereicherten Entspannungsgasstroms,
(e) einen Lagertank, eine Leitung zum Einleiten des entspannten Rohmethanolstroms in den Lagertank, eine Leitung zum Einleiten eines Inertisierungsgases und eine Leitung zum Ausleiten eines mit Methanol beladenen Inertisierungsgasstroms aus dem Lagertank, eine Leitung zum Ausleiten des Rohmethanols,
(f) eine als Gegenstromwäscher ausgestaltete Inertisierungsgaswaschvorrichtung, eine Leitung zum Einleiten eines mit Methanol beladenen Inertisierungsgasstroms in die Inertisierungsgaswaschvorrichtung, eine Leitung zum Einleiten eines Wasserstroms als Waschmittel in die Inertisierungsgaswaschvorrichtung, eine Leitung zum Ausleiten eines an Methanol abgereicherten Inertisierungsgasstroms und eine Leitung zum Ausleiten eines mit Methanol beladenen Wasserstroms aus der Inertisierungsgaswaschvorrichtung.

11. Vorrichtung nach Anspruch 10, ferner umfassend eine Wasserstoffwiedergewinnungsvorrichtung und eine Leitung zum Einleiten eines an Methanol abgereicherten Purgegasstroms in die Wasserstoffwiedergewinnungsvorrichtung.

12. Vorrichtung nach Anspruch 10, ferner umfassend eine Leitung zum Zuführen des in Verfahrensschritt (d) erhaltenen, mit Methanol beladenen Wasserstroms zum Entspannungsbehälter.

13. Vorrichtung nach Anspruch 10, ferner umfassend eine Leitung zum Zuführen des in Verfahrensschritt (d) erhaltenen, mit Methanol beladenen Wasserstroms als Waschmittel zu der Entspannungsgaswaschvorrichtung.

14. Vorrichtung nach Anspruch 10, ferner umfassend eine Destillationsvorrichtung und eine Leitung zum Zuführen des in Verfahrensschritt (e) erhaltenen, entspannten Rohmethanolstroms zu der Destillationsvorrichtung.

15. Vorrichtung nach Anspruch 10, ferner umfassend eine Leitung zum Zuführen mindestens eines Teils des in Verfahrensschritt (g) erhaltenen, an Methanol abgereicherten Entspannungsgasstroms als Inertisierungsgas in den Lagertank.

16. Vorrichtung nach Anspruch 10, ferner umfassend eine Leitung zum Zuführen mindestens eines Teils des in Verfahrensschritt (j) erhaltenen, mit Methanol beladenen Wasserstroms als Waschmittel zu der Entspannungsgaswaschvorrichtung.

17. Vorrichtung nach Anspruch 10, ferner umfassend eine Leitung zum Zuführen mindestens eines Teils des in Verfahrensschritt (j) erhaltenen, mit Methanol beladenen Wasserstroms zum Entspannungsbehälter.

18. Vorrichtung nach einem der vorgenannten Ansprüche in einer Anlage zur Herstellung von Methanol durch Umsetzung kohlebasierten Synthesegases.

## Claims

1. Process for recovering methanol from process offgases from a methanol synthesis, comprising the following process steps:
(a) reacting a synthesis gas stream under methanol synthesis conditions in at least one methanol synthesis reactor arranged in a synthesis gas circuit for unconverted synthesis gas,
(b) discharging a liquid crude methanol stream from the synthesis gas circuit and discharging a purge gas stream from the synthesis gas circuit, wherein the purge gas stream is laden with methanol vapour,
(c) introducing the purge gas stream into a purge gas scrubbing apparatus, contacting the purge gas stream in the purge gas apparatus with a water stream as scrubbing medium which is passed through the purge gas scrubbing apparatus countercurrently to the purge gas stream, wherein the water stream absorbs at least part of the methanol from the purge gas stream,
(d) discharging a methanol-laden water stream and a methanol-depleted purge gas stream from the purge gas scrubbing apparatus,
(e) introducing the liquid crude methanol stream into a decompression vessel in which the pressure of the liquid crude methanol stream is reduced to obtain a methanol-laden decompression gas stream and a decompressed crude methanol stream,
(f) introducing the decompression stream into a decompression gas scrubbing apparatus, contacting the decompression gas stream in the decompression gas scrubbing apparatus with a water stream as scrubbing medium which is passed through the decompression gas scrubbing apparatus countercurrently to the decompression gas stream, wherein the water stream absorbs at least part of the methanol from the decompression gas stream,
(g) discharging a methanol-laden water stream and a methanol-depleted decompression gas stream from the decompression gas scrubbing apparatus, wherein the methanol-laden water stream is added to the decompressed crude methanol stream,
(h) introducing the decompressed crude methanol stream into a storage tank in which it is stored until further processing or further use and covered by an inertization gas which becomes laden with methanol,
(i) discharging a methanol-laden inertization gas stream from the storage tank and introducing it into an inertization gas scrubbing apparatus, contacting the inertization gas stream in the inertization gas scrubbing apparatus with a water stream as scrubbing medium which is passed through the inertization gas scrubbing apparatus countercurrently to the inertization gas stream, wherein the water stream absorbs at least part of the methanol from the inertization gas stream,
(j) discharging a methanol-laden water stream and a methanol-depleted inertization gas stream from the inertization gas scrubbing apparatus.

2. Process according to claim 1, **characterized in that** the methanol-depleted purge gas stream obtained in step (d) is supplied to a hydrogen recovery apparatus.

3. Process according to claim 1, **characterized in that** the methanol-laden water stream obtained in step (d) is at least partly supplied to the decompression vessel.

4. Process according to claim 1, **characterized in that** the methanol-laden water stream obtained in step (d) is at least partly used as scrubbing medium in the decompression gas scrubbing apparatus.

5. Process according to claim 1, **characterized in that** at least part of the decompressed crude methanol stream obtained in step (e) is supplied to a distillation apparatus without intermediate storage in the storage tank.

6. Process according to claim 1, **characterized in that** at least part of the methanol-depleted decompression gas stream obtained in step (g) is introduced into the storage tank as inertization gas.

7. Process according to claim 1, **characterized in that** at least part of the methanol-laden water stream obtained in step (j) is used as scrubbing medium in the decompression gas scrubbing apparatus.

8. Process according to claim 1, **characterized in that** at least part of the methanol-laden water stream obtained in step (j) is supplied to the decompression vessel.

9. Process according to any of the preceding claims, **characterized in that** it is used for recovery of methanol from process offgases from a plant for methanol synthesis in which methanol is produced from coal-based synthesis gas.

10. Apparatus for recovery of methanol from process offgases from methanol synthesis, comprising the following constituents and assemblies:
(a) a methanol synthesis reactor arranged in a synthesis gas circuit for unconverted synthesis gas,
(b) a conduit for discharging a liquid crude methanol stream from the synthesis gas circuit and a conduit for discharging a purge gas stream from the synthesis gas circuit, wherein the purge gas stream is laden with methanol vapour,
(c) a purge gas scrubbing apparatus configured as a countercurrent scrubber, a conduit for introducing the purge gas stream into a purge gas scrubbing apparatus, a conduit for supplying a water stream as scrubbing medium, a conduit for discharging a methanol-laden water stream and a conduit for discharging a methanol-depleted purge gas stream from the purge gas scrubbing apparatus,
(d) a decompression vessel in which the pressure of the liquid crude methanol stream is reduced, a conduit for introducing the liquid crude methanol stream into the decompression vessel, a decompression gas scrubbing apparatus configured as a countercurrent scrubber and connected to the decompression vessel, a conduit for introducing a water stream as scrubbing medium into the decompression gas scrubbing apparatus, a conduit for discharging a decompressed crude methanol stream and a conduit for discharging a methanol-depleted decompression gas stream,
(e) a storage tank, a conduit for introducing the decompressed crude methanol stream into the storage tank, a conduit for introducing an inertization gas and a conduit for discharging a methanol-laden inertization gas stream from the storage tank, a conduit for discharging the crude methanol,
(f) an inertization gas scrubbing apparatus configured as a countercurrent scrubber, a conduit for introducing a methanol-laden inertization gas stream into the inertization gas scrubbing apparatus, a conduit for introducing a water stream as scrubbing medium into the inertization gas scrubbing apparatus, a conduit for discharging a methanol-depleted inertization gas stream and a conduit for discharging a methanol-laden water stream from the inertization gas scrubbing apparatus.

11. Apparatus according to claim 10, further comprising a hydrogen recovery apparatus and a conduit for introducing a methanol-depleted purge gas stream into the hydrogen recovery apparatus.

12. Apparatus according to claim 10, further comprising a conduit for supplying the methanol-laden water stream obtained in process step (d) to the decompression vessel.

13. Apparatus according to claim 10, further comprising a conduit for supplying the methanol-laden water stream obtained in process step (d) to the decompression gas scrubbing apparatus as scrubbing medium.

14. Apparatus according to claim 10, further comprising a distillation apparatus and a conduit for supplying the decompressed crude methanol stream obtained in step (e) to the distillation apparatus.

15. Apparatus according to claim 10, further comprising a conduit for supplying at least part of the methanol-depleted decompression gas stream obtained in process step (g) into the storage tank as inertization gas.

16. Apparatus according to claim 10, further comprising a conduit for supplying at least part of the methanol-laden water stream obtained in process step (j) to the decompression gas scrubbing apparatus as scrubbing medium.

17. Apparatus according to claim 10, further comprising a conduit for supplying at least part of the methanol-laden water stream obtained in process step (j) to the decompression vessel.

18. Apparatus according to any of the preceding claims in a plant for producing methanol by reaction of coal-based synthesis gas.

## Revendications

1. Procédé de récupération de méthanol à partir de gaz d'échappement de procédé de la synthèse de méthanol, comprenant les étapes de procédé suivantes :
(a) mise en réaction d'un courant de gaz de synthèse dans des conditions de synthèse de méthanol dans au moins un réacteur de synthèse de méthanol, qui est agencé dans un circuit de gaz de synthèse pour le gaz de synthèse non réagi,
(b) déchargement d'un courant de méthanol brut liquide hors du circuit de gaz de synthèse et le déchargement d'un courant de gaz de purge hors du circuit de gaz de synthèse, le courant de gaz de purge étant chargé avec de la vapeur de méthanol,
(c) introduction du courant de gaz de purge dans un dispositif de lavage du gaz de purge, mise en contact du courant de gaz de purge dans le dispositif de lavage du gaz de purge avec un courant d'eau en tant qu'agent de lavage, qui est mis en circulation à contre-courant du courant de gaz de purge dans le dispositif de lavage du gaz de purge, le courant d'eau absorbant au moins une partie du méthanol hors du courant de gaz de purge,
(d) déchargement d'un courant d'eau chargé avec du méthanol et d'un courant de gaz de purge appauvri en méthanol hors du dispositif de lavage du gaz de purge,
(e) introduction du courant de méthanol brut liquide dans un contenant de détente, dans lequel la pression du courant de méthanol brut liquide est réduite, un courant de gaz de détente chargé avec du méthanol et un courant de méthanol brut détendu étant obtenus,
(f) introduction du courant de gaz de détente dans un dispositif de lavage du gaz de détente, mise en contact du courant de gaz de détente dans le dispositif de lavage du gaz de détente avec un courant d'eau en tant qu'agent de lavage, qui est mis en circulation à contre-courant du courant de gaz de détente dans le dispositif de lavage du gaz de détente, le courant d'eau absorbant au moins une partie du méthanol du courant de gaz de détente,
(g) déchargement d'un courant d'eau chargé avec du méthanol et d'un courant de gaz de détente appauvri en méthanol hors du dispositif de lavage du gaz de détente, le courant d'eau chargé avec du méthanol étant ajouté au courant de méthanol brut détendu,
(h) introduction du courant de méthanol brut détendu dans une cuve de stockage, dans laquelle il est stocké jusqu'à sa transformation ultérieure ou son utilisation ultérieure et recouvert par un gaz d'inertisation qui est chargé avec du méthanol,
(i) déchargement d'un courant de gaz d'inertisation chargé avec du méthanol hors de la cuve de stockage et l'introduction dans un dispositif de lavage du gaz d'inertisation, mise en contact du courant de gaz d'inertisation dans le dispositif de lavage du gaz d'inertisation avec un courant d'eau en tant qu'agent de lavage, qui est mis en circulation à contre-courant du courant de gaz d'inertisation dans le dispositif de lavage du gaz d'inertisation, le courant d'eau absorbant au moins une partie du méthanol hors du courant de gaz d'inertisation,
(j) déchargement d'un courant d'eau chargé avec du méthanol et d'un courant de gaz d'inertisation appauvri en méthanol du dispositif de lavage hors du gaz d'inertisation.

2. Procédé selon la revendication 1, **caractérisé en ce que** le courant de gaz de purge appauvri en méthanol obtenu à l'étape (d) est introduit dans un dispositif de récupération de l'hydrogène.

3. Procédé selon la revendication 1, **caractérisé en ce que** le courant d'eau chargé avec du méthanol obtenu à l'étape (d) est au moins partiellement introduit dans le contenant de détente.

4. Procédé selon la revendication 1, **caractérisé en ce que** le courant d'eau chargé avec du méthanol obtenu à l'étape (d) est au moins partiellement utilisé en tant qu'agent de lavage dans le dispositif de lavage du gaz de détente.

5. Procédé selon la revendication 1, **caractérisé en ce qu'**au moins une partie du courant de méthanol brut détendu obtenu à l'étape (e) est introduite dans la cuve de stockage d'un dispositif de distillation sans stockage intermédiaire.

6. Procédé selon la revendication 1, **caractérisé en ce qu'**au moins une partie du courant de gaz de détente appauvri en méthanol obtenu à l'étape (g) est introduite dans la cuve de stockage en tant que gaz d'inertisation.

7. Procédé selon la revendication 1, **caractérisé en ce qu'**au moins une partie du courant d'eau chargé avec du méthanol obtenu à l'étape (j) est utilisée en tant qu'agent de lavage dans le dispositif de lavage du gaz de détente.

8. Procédé selon la revendication 1, **caractérisé en ce qu'**au moins une partie du courant d'eau chargé avec du méthanol obtenu à l'étape (j) est introduite dans le contenant de détente.

9. Procédé selon l'une quelconque des revendications précédentes, **caractérisé en ce qu'**il est utilisé pour la récupération de méthanol à partir de gaz d'échappement de procédé d'une unité pour la synthèse de méthanol, dans laquelle du méthanol est fabriqué à partir d'un gaz de synthèse à base de charbon.

10. Dispositif pour la récupération de méthanol à partir de gaz d'échappement de procédé de la synthèse de méthanol, comprenant les constituants et sous-composants suivants :
(a) un réacteur de synthèse de méthanol, qui est agencé dans un circuit de gaz de synthèse pour le gaz de synthèse non réagi,
(b) une conduite pour le déchargement d'un courant de méthanol brut liquide hors du circuit de gaz de synthèse et une conduite pour le déchargement d'un courant de gaz de purge hors du circuit de gaz de synthèse, le courant de gaz de purge étant chargé avec de la vapeur de méthanol,
(c) un dispositif de lavage du gaz de purge configuré sous la forme d'un épurateur à contre-courant, une conduite pour l'introduction du courant de gaz de purge dans un dispositif de lavage du gaz de purge, une conduite pour l'introduction d'un courant d'eau en tant qu'agent de lavage, une conduite pour le déchargement d'un courant d'eau chargé avec du méthanol et une conduite pour le déchargement d'un courant de gaz de purge appauvri en méthanol du dispositif de lavage du gaz de purge,
(d) un contenant de détente, dans lequel la pression du courant de méthanol brut liquide est réduite, une conduite pour l'introduction du courant de méthanol brut liquide dans le contenant de détente, un dispositif de lavage du gaz de détente configuré sous la forme d'un épurateur à contre-courant, raccordé avec le contenant de détente, une conduite pour l'introduction d'un courant d'eau en tant qu'agent de lavage dans le dispositif de lavage du gaz de détente, une conduite pour le déchargement d'un courant de méthanol brut détendu et une conduite pour le déchargement d'un courant de gaz de détente appauvri en méthanol,
(e) une cuve de stockage, une conduite pour l'introduction du courant de méthanol brut détendu dans la cuve de stockage, une conduite pour l'introduction d'un gaz d'inertisation et une conduite pour le déchargement d'un courant de gaz d'inertisation chargé avec du méthanol hors de la cuve de stockage, une conduite pour le déchargement du méthanol brut,
(f) un dispositif de lavage du gaz d'inertisation configuré sous la forme d'un épurateur à contre-courant, une conduite pour l'introduction d'un courant de gaz d'inertisation chargé avec du méthanol dans le dispositif de lavage du gaz d'inertisation, une conduite pour l'introduction d'un courant d'eau en tant qu'agent de lavage dans le dispositif de lavage du gaz d'inertisation, une conduite pour le déchargement d'un courant de gaz d'inertisation appauvri en méthanol et une conduite pour le déchargement d'un courant d'eau chargé avec du méthanol hors du dispositif de lavage du gaz d'inertisation.

11. Dispositif selon la revendication 10, comprenant en outre un dispositif de récupération de l'hydrogène et une conduite pour l'introduction d'un courant de gaz de purge appauvri en méthanol dans le dispositif de récupération de l'hydrogène.

12. Dispositif selon la revendication 10, comprenant en outre une conduite pour l'introduction du courant d'eau chargé avec du méthanol obtenu à l'étape de procédé (d) dans le contenant de détente.

13. Dispositif selon la revendication 10, comprenant en outre une conduite pour l'introduction du courant d'eau chargé avec du méthanol obtenu à l'étape de procédé (d) en tant qu'agent de lavage dans le dispositif de lavage du gaz de détente.

14. Dispositif selon la revendication 10, comprenant en outre un dispositif de distillation et une conduite pour l'introduction du courant de méthanol brut détendu obtenu à l'étape de procédé (e) dans le dispositif de distillation.

15. Dispositif selon la revendication 10, comprenant en outre une conduite pour l'introduction d'au moins une partie du courant de gaz de détente appauvri en méthanol obtenu à l'étape de procédé (g) en tant que gaz d'inertisation dans la cuve de stockage.

16. Dispositif selon la revendication 10, comprenant en outre une conduite pour l'introduction d'au moins une partie du courant d'eau chargé avec du méthanol obtenu à l'étape de procédé (j) en tant qu'agent de lavage dans le dispositif de lavage du gaz de détente.

17. Dispositif selon la revendication 10, comprenant en outre une conduite pour l'introduction d'au moins une partie du courant d'eau chargé avec du méthanol obtenu à l'étape de procédé (j) dans le contenant de détente.

18. Dispositif selon l'une quelconque des revendications précédentes dans une unité pour la fabrication de méthanol par mise en réaction d'un gaz de synthèse à base de charbon.
